# EUROPÄISCHE PATENTSCHRIFT

(11) **EP 0 502 253 B1**
(45) Veröffentlichungstag und Bekanntmachung des Hinweises auf die Patenterteilung: **15.03.1995**
(21) Anmeldenummer: 91121045.8
(22) Anmeldetag: 09.12.1991
(51) Int. Cl.: C07C 39/17, C07C 37/20

(54) **Verfahren zur Herstellung von 9,9-Bis-(4-hydroxyphenyl-)fluoren**
Process for the preparation of 9,9-bis-(4-hydroxyphenyl)fluorene
Procédé de préparation de 9,9-bis-(4-hydroxyphenyl-)fluorène

(30) Priorität: 07.03.1991 DE 4107242
(43) Veröffentlichungstag der Anmeldung: 09.09.1992
(73) Patentinhaber: RÜTGERSWERKE AKTIENGESELLSCHAFT, 60326 Frankfurt (DE)
(72) Erfinder: Orth, Winfried, Dr., W-6733 Hassloch/Pfalz (DE); Pastorek, Emmerich, Dr., W-6944 Hemsbach a. d. Bergstrasse (DE); Weiss, Wolfgang, Dr., W-6803 Neckarhausen (DE); Kleffner, Hans Werner, Dr., W-6719 Battenberg/Pfalz (DE)

(56) Entgegenhaltungen:
- EP-A- 0 180 133
- US-A- 3 546 165
- PATENT ABSTRACTS OF JAPAN, vol. 12, no. 103 (C-485)(2950), 5. April 1988; & JP-A-62 230 741

## Beschreibung

Die Erfindung betrifft ein verbessertes Verfahren zur Herstellung von 9,9-Bis-(4-hydroxyphenyl-)fluoren durch Kondensation von Phenol und Fluorenon und das anschließende Verfahren zur Isolierung des Reinprodukts.

In der Kunststoffindustrie werden solche Bisphenole, insbesondere als Monomere, in großem Umfang in Polykondensationsprozessen eingesetzt. Hergestellt werden daraus beispielsweise Polyesterharze und Kunststoffe mit besonders guten hitzebeständigen Eigenschaften, die für Isolierungen elektrischer Leiter oder für hochtemperaturbeständige Überzüge verwendet werden können.

Kunststoffe mit diesen vorteilhaften Eigenschaften können jedoch nur erhalten werden, wenn zu ihrer Herstellung besonders reine Bisphenole verwendet werden. Der wirtschaftlichen Bedeutung entsprechend wurden daher zahlreiche Versuche zur Verbesserung bekannter Synthesewege und zur Reinigung des Reaktionsprodukts unternommen.

Eine zentrale Rolle spielt in diesem Zusammenhang das von Morgan beschriebene Verfahren (P. W. Morgan, Makromolecules, 3, 536 (1970); (US 3,546,165 A1)), an das sich fast alle späteren Verfahren anlehnen. In der Kondensationsreaktion werden danach Fluorenon und Phenol im molaren Verhältnis von 1 : 4 eingesetzt, so daß Phenol während der Umsetzung gleichzeitig als Reaktand und Lösungsmittel fungiert. Die Reaktion selbst läuft in Gegenwart von β-Mercaptopropionsäure oder Mercaptoessigsäure unter Einleitung von trockenem HCl-Gas bei einer Temperatur zwischen 140 und 150 °C ab. Die hohen Temperaturen sind bei diesem Mischungsverhältnis der Reaktanden notwendig, damit die Masse rührfähig bleibt. Sie führen jedoch, wie spätere Versuche ergeben haben, zur Bildung von unerwünschten Nebenprodukten.

Die von Morgan im Anschluß an die Kondensationsreaktion vorgeschlagene Verdünnung mit Wasser oder die Wasserdampfdestillation sind zur Aufarbeitung des Reaktionsgemischs im technischen Maßstab ungeeignet. Zwar bildet sich bei der Verdünnung mit Wasser tatsächlich eine weiße Masse, jedoch ist sie kompakt, klebrig und in größeren Mengen nicht mehr zu handhaben. Auch durch eine Wasserdampfdestillation wird die gleiche klebrige Masse erhalten. Außerdem wird offensichtlich gerade durch die Behandlung mit heißem Wasser eine zusätzliche Verfärbung des Reaktionsprodukts hervorgerufen und eine Auskristallisation des Produktes verhindert.

Nach der Beschreibung von Morgan wird das abgetrennte Produkt in alkalischer Lösung aufgelöst und durch Zutropfen von Salzsäure erneut ausgefällt. Nach einer Umkristallisation aus Toluol wird 9,9-Bis-(4-hydroxyphenyl-)fluoren als weißes kristallines Produkt mit einem Schmelzpunkt von 224 bis 225 °C in einer Ausbeute von 46 bis 56 % der Theorie erhalten.

Eigene Versuche, die oben beschriebene weiße Masse in alkalischer Lösung zu lösen, gestalteten sich jedoch aufwendig und langwierig.

Ziel späterer Verfahren war es daher, einerseits die Ausbeute zu steigern, andererseits die Aufarbeitung zu vereinfachen.

Zur Ausbeutesteigerung wurden im Laufe der Zeit verschiedenste im Verfahren einsetzbare Katalysatoren ausprobiert. Dazu gehören ionisierbare Schwefelverbindungen wie z. B. Schwefelmonochlorid, Schwefelwasserstoff, verschiedenste Mercaptoverbindungen oder Alkalisulfide, die mit Säuren unter Bildung von Schwefelwasserstoff reagieren. Eingesetzt wurden auch Friedel-Crafts-Katalysatoren wie z. B. ZnCl₂, CaCl₂, AlCl₃ oder SnCl₄, die in Gegenwart von HCl wirksam sind.

Es stellte sich jedoch heraus, daß nicht nur die Wahl des Katalysators die Ausbeutemenge und die Produktqualität entscheidend beeinflussen, sondern insbesondere die Reaktionstemperatur und das molare Verhältnis der Reaktanden zueinander. Hohe Ausbeuten konnten daher sowohl mit Friedel-Crafts-Katalysatoren als auch mit der durch Morgan vorbeschriebenen Mercaptopropionsäure erzielt werden, wenn die Reaktionstemperatur unter 100 °C, insbesondere zwischen 30 und 90 °C gehalten und Phenol in der vier- bis achtfachen molaren Menge bezogen auf das Fluorenon eingesetzt wurde.

In diesem Temperaturbereich wird auch die Kondensationsreaktion in dem in der Offenlegungsschrift DE-OS 34 39 484 beschriebenen Verfahren durchgeführt. Fluorenon und Phenol werden dabei im molaren Verhältnis von 1 : 4 bis 1 : 6 in Gegenwart von β-Mercaptopropionsäure und konzentrierter Schwefelsäure umgesetzt. Durch das sich anschließende Aufarbeitungsverfahren wird nach den Angaben der Anmelderin eine Rohausbeute von etwa 97 bis 98 % erhalten. Darin ist jedoch noch ein hoher Anteil unerwünschter Nebenprodukte enthalten, die durch Dimerisierung und Substitution entstanden sind. Die weitere Aufarbeitung erfolgt, indem nach beendeter Kondensationsreaktion Methanol hinzugefügt und erst anschließend in kaltes Wasser gegossen wird. Auch hier scheidet sich die ölige Masse ab. Die überstehende wäßrige Methanol-Schwefelsäure-Phenol-Lösung wird abgetrennt und der Rückstand noch zweimal mit Wasser gewaschen und mit Ammoniumcarbonatlösung neutralisiert.

Noch anhaftendes Phenol wird durch mehrmaliges Aufkochen mit Wasser entfernt. Nach dem Trocknen und nochmaliger Umkristallisation mit Isopropylalkohol wird ein Produkt mit einem Schmelzpunkt von 223 °C erhalten.

Dieses Verfahren besitzt jedoch gravierende Nachteile. Bei der Aufarbeitung des Reaktionsprodukts fallen sehr große Mengen mit Phenol und Schwefelsäure verunreinigten Wassers an, die aufgrund der Gewässerschutzverordnungen einer besonderen Aufarbeitung zugeführt werden müssen. Außerdem ist auch bei diesem Verfahren das nach der Wasserzugabe anfallende, verklumpte Rohprodukt schwer zu handhaben. Es läßt sich nur schwer abfiltrieren und zur weiteren Reinigung waschen und neutralisieren.

Aufgabe der Erfindung ist es daher, ein preiswertes leicht durchführbares Verfahren zur Herstellung von 9,9-Bis-(4-hydroxyphenyl-)fluoren in hoher Ausbeute und Reinheit zur Verfügung zu stellen, das es erlaubt, zur Produktreinigung verwendete Lösungsmittel in einfacher Weise abzutrennen, so daß sie möglichst erneut im Verfahren eingesetzt werden können.

Die Lösung der Aufgabe erfolgt durch ein Verfahren gemäß der Ansprüche 1 bis 5 und seiner Ausgestaltung gemäß der Ansprüche 6 bis 10.

Es wurde gefunden, daß die im Reaktionsgemisch enthaltene Salzsäure gemeinsam mit dem entstandenen Reaktionswasser leicht unter vermindertem Druck abdestilliert werden kann, wenn nach der von Morgan beschriebenen Methode gearbeitet wird. Dieses hat den Vorteil, daß unter wesentlich milderen Bedingungen gearbeitet werden kann, und sich weniger unerwünschte Nebenprodukte durch Dimerisierung und Substitution bilden, als wenn mit konzentrierter Schwefelsäure gearbeitet wird.

Gleichzeitig können dadurch Nachteile vermieden werden, die bei der Produktaufarbeitung und durch die dabei anfallenden Lösungsmittelmengen auftreten, wenn in Gegenwart von Schwefelsäure oder von Friedel-Crafts-Katalysatoren gearbeitet wird.

Wie sich gezeigt hat, läuft die Kondensationsreaktion von Fluoren und Phenol in Gegenwart von HCl bereits bei 30 °C in ausreichender Geschwindigkeit ab. Bevorzugt wird bei Temperaturen zwischen 50 und 60 °C gearbeitet, da in diesem Bereich die Bildung von Nebenprodukten niedrig ist, und die Reaktionszeit bis zum vollständigen Umsatz von Fluorenon annehmbar kurz ist.

Werden die Reaktanden Fluorenon und Phenol, wie bei Morgan angegeben, im molaren Verhältnis von 1 : 4 zur Reaktion eingesetzt, verfestigt sich gegen Ende die Reaktionsmasse. Wird dagegen Phenol in der sechs- bis achtfachen molaren Menge, bezogen auf Fluorenon, eingesetzt, bleibt die Masse durchgehend rührfähig.

Nach der Reaktion läßt sich zwar das Reaktionswasser gemeinsam mit gelöster Salzsäure unter vermindertem Druck abdestillieren, für das im Überschuß eingesetzte Phenol ist das jedoch nicht zu empfehlen. Wird es völlig abdestilliert, erhält man eine kompakte Masse als Destillationsrückstand, die nicht mehr weiter zu verarbeiten ist.

Überraschenderweise wurde nun gefunden, daß dieses umgangen werden kann, indem nach der Kondensationsreaktion und dem Abdestillieren des salzsäurehaltigen Reaktionswassers ein Alkyl- oder ein Aralkylnitril zu dem verbliebenen Reaktionsgemisch gegeben wird. Das Reaktionsprodukt bildet mit dem Nitril ein in Phenol schwer lösliches kristallines Addukt und kann leicht abgetrennt werden.

Das im Überstand enthaltene Phenol kann nach der Abtrennung der Kristalle von dem evtl. überschüssigen Nitril auf an sich bekannte Weise abgetrennt und in den Prozeß zurückgeführt werden.

Auf diese Weise entfällt die sonst übliche Wasserwäsche oder Wasserdampfdestillation zur Phenol- und Säureabtrennung, so daß die Abscheidung des Produkts als klebrige kompakte Masse vermieden werden kann.

Als weiterer Vorteil erweist sich, daß im Prinzip außer äquimolaren Mengen Nitril kein weiteres Lösungsmittel zur Abtrennung des Reaktionsprodukts benötigt wird. In einem einfachen Verfahrensschritt läßt sich das gebildete Produkt zu über 90 % aus dem Reaktionsgemisch abtrennen. Wird dann die abgetrennte Phenolphase erneut zur Umsetzung eingesetzt, läßt sich noch ein großer Teil des im ersten Schritt nicht isolierten Produkts gewinnen.

Zur Reinigung des Addukts ist eine einfache Umkristallisation aus einem bei Raumtemperatur flüssigen Nitril oder aus einem unpolaren Lösungsmittel geeignet.

Auch die Isolierung des 9,9-Bis-(4-hydroxyphenyl-)fluorens aus dem Addukt gestaltet sich sehr einfach. Addukte, die durch Addition niedrigsiedender Nitrile gebildet werden, lassen sich durch einfache thermische Behandlung aufspalten. Höher siedende Nitrile, also solche mit einem Siedepunkt oberhalb von 130 °C, lassen sich dagegen leichter und ökonomischer durch Erwärmen in Gegenwart von Wasser abspalten.

Bei der Produktabtrennung als Nitriladdukt ist es von großem Vorteil, daß die Aufspaltung bzw. das Trocknen entfallen kann, wenn 9,9-Bis-(4-hydroxyphenyl-)fluoren z. B. zur Herstellung von polymeren Werkstoffen verwendet werden soll. 9,9-Bis-(4-hydroxyphenyl-)fluoren ist ein potentielles Allergen. In reiner, getrockneter Form ist es ein fein kristallines und staubendes Produkt. Die Addukte fallen jedoch vorwiegend als rieselfähige nicht staubende Kristalle an, die gefahrlos zu handhaben sind.

Da einerseits die Nitrile bei bestimmten Polymerisationsreaktionen nicht stören, ja sogar als willkommenes Lösungsmittel dienen können, kann in diesen Fällen auf die Isoliereung des Reinprodukts verzichtet werden und sogar das durch Lösungsmittel oder Nitril feuchte Addukt weiterverwendet werden. Bei Umsetzungen mit Epichlorhydrin wirken geringe Mengen Wasser nicht störend, so daß auch wasserfeuchtes isoliertes Produkt weiterverwendet werden kann.

Zur Abtrennung von 9,9-Bis-(4-hydroxyphenyl-)fluoren vom Reaktionsgemisch können Nitrile der Formel R-CN zugesetzt werden, wobei R ein verzweigter oder unverzweigter, gesättigter Alkyl-, Aryl- oder Aralkylrest mit 1 bis 15 C-Atomen oder einem Arylalkylether ist. Verwendet werden können aber auch Dinitrile der Formel NC-A-CN, wobei A eine verzweigte oder unverzweigte, gesättigte Alkylkette mit 1 bis 6 C-Atomen oder eine substituierte oder unsubstituierte Arylgruppe ist. Oktansäurenitril, Acrylnitril, sowie Cyanessigsäureessigester ergeben jedoch keine Addukte.

Geeignete Nitrile sind z. B.
Acetonitril,
Propionsäurenitril,
n-Capronsäurenitril,
Caprinsäurenitril,
Palmitinsäurenitril,
Trimethylacetonitril,
Malonsäuredinitril,
Bernsteinsäuredinitril,
Glutarsäuredinitril,
Adipinsäuredinitril,
Hexamethylendinitril,
Benzonitril,
o- und p-Tolunitril,
Phenoxybuttersäurenitril,
Anthranilsäurenitril oder
β-Naphthonitril.

Vorzugsweise wird die Abtrennung mit niedrigsiedenden, gesättigten Alkylnitrilen durchgeführt, wenn das Reinprodukt isoliert werden soll. Bevorzugt werden in diesem Fall Acetonitril oder Propionsäurenitril verwendet, die durch einfaches Abdestillieren vom überschüssigen Phenol abgetrennt werden können.

Wird 9,9-Bis-(4-hydroxyphenyl-)fluoren zur Herstellung von Polyestern verwendet, erweist es sich als vorteilhaft, die Abtrennung mit Hilfe von hochsiedenden Dinitrilen durchzuführen und die Addukte direkt zur Polymerisation einzusetzen. Während der Reaktion werden die Dinitrile aus den Addukten freigesetzt und wirken als Lösungsmittel. Vorzugsweise werden zu diesem Zweck Dinitrile mit gesättigten, unverzweigten Alkylketten mit 3 bis 6 C-Atomen verwendet. Besonders bevorzugt wird mit Adipinsäuredinitril gearbeitet.

Zur Durchführung des Verfahrens werden Fluorenon und Phenol z. B. im molaren Verhältnis 1 : 6 im Reaktionsgefäß vorgelegt und β-Mercaptopropionsäure als Katalysator in einer Menge von etwa 0,01 Mol pro Mol eingesetzten Fluorenons hinzugefügt. Dieses Gemisch wird auf eine Temperatur von 50 bis 60 °C erwärmt. Unter Rühren wird trockenes Chlorwasserstoffgas in einer Menge von etwa 0,3 bis 0,45 mol pro Mol eingesetzten Fluorenons eingeleitet und noch 2 bis 6 h nachgerührt.

Anschließend wird bei vermindertem Druck und Temperaturen zwischen 40 und 90 °C das entstandene Reaktionswasser gemeinsam mit der gelösten Salzsäure abdestilliert.

Zur Adduktbildung wird nun das Nitril in der zwei- bis fünffachen molaren Menge, bezogen auf das eingesetzte Fluorenon, zu dem Destillationsrückstand gegeben. Nachdem sich alles gelöst hat, wird das Gemisch abgekühlt. Das gebildete Addukt fällt dabei weiß, kristallin aus und kann in an sich bekannter Weise abgetrennt werden. Mit Hilfe von Acetonitril kann das Reaktionsprodukt nach dem Trocknen im Vakuum in einem einzigen Verfahrensschritt bereits mit einer Reinheit von etwa 98 % gewonnen werden. Durch Umkristallisieren aus weiterem Nitril kann die Reinheit auf 99,9 % gesteigert werden.

Werden Dinitrile, wie z. B. Adipinsäuredinitril, zur Adduktbildung verwendet, kann dieses Addukt direkt ohne Auftrennung zur Herstellung von Polyestern eingesetzt werden.

Das abgetrennte Addukt kann aber auch auf einfache Weise aufgespalten werden, indem es mit einer ausreichenden Menge Wasser (etwa 1 l Wasser pro eingesetztem Mol Fluorenon) aufgeschlämmt und gerührt und ggf. unter Rühren auf eine Temperatur bis 90 °C erwärmt wird. Bei Raumtemperatur fällt das 9,9-Bis-(4-hydroxyphenyl-)fluoren kristallin aus und kann abfiltriert werden. Nach dem Trocknen und Umkristallisieren wird 9,9-Bis-(4-hydroxyphenyl-)fluoren mit einer Reinheit von 99,7 bis 99,9 % erhalten.

Die folgenden Beispiele sollen zur näheren Erläuterung der Erfindung dienen, diese jedoch nicht auf die Beispiele beschränken.

### Beispiel 1

In eine Schmelze von 180 g 9-Fluorenon (1 mol), 565 g Phenol (6 mol) und 1 g Mercaptopropionsäure leitet man bei 55 °C innerhalb von 6 h 14 g Chlorwasserstoff (0,38 mol) ein und rührt noch 2 h bei 55 °C nach. Anschließend wird das gebildete Reaktionswasser gemeinsam mit der enthaltenen Salzsäure abdestilliert. Der Destillationsrückstand wird nun unter Rühren in 500 ml Acetonitril aufgelöst und anschließend auf 0 °C abgekühlt. Dabei fällt ein weißes kristallines Produkt aus. Es handelt sich um ein Addukt aus 9,9-Bis-(4-hydroxyphenyl-)fluoren und Acetonitril. Nach dem Abfiltrieren wird dieses Addukt noch viermal mit je 150 ml Acetonitril nachgewaschen.

### Beispiel 2

9,9-Bis-(4-hydroxyphenyl-)fluoren wird aus dem Acetonitril-Addukt freigesetzt, indem die im Beispiel 1 erhaltenen weißen Kristalle mit 1.000 ml Wasser aufgeschlämmt und etwa 30 min gerührt werden. Anschließend wird das weiße, fein kristalline 9,9-Bis-(4-hydroxyphenyl-)fluoren abfiltriert und getrocknet.

9,9-Bis-(4-hydroxyphenyl-)fluoren wird so in einer Ausbeute von 80 % der Theorie mit einer Reinheit von 98 % erhalten.

Wird das abgetrennte Addukt vor der Isolierung des Fluorenderivats noch einmal aus Acetonitril umkristallisiert, wird es mit einer Reinheit von 99,9 % erhalten.

### Beispiel 3

9,9-Bis-(4-hydroxyphenyl-)fluoren wird aus dem Acetonitril-Addukt freigesetzt, indem die im Beispiel 1 erhaltenen weißen Kristalle im Vakuum bei 110 bis 120 °C getrocknet werden.

9,9-Bis-(4-hydroxyphenyl-)fluoren wird so in einer Ausbeute von 80 % der Theorie mit einer Reinheit von 98 % erhalten.

Wird das abgetrennte Addukt vor der Isolierung des Fluorenderivats noch einmal aus Acetonitril umkristallisiert, wird es mit einer Reinheit von 99,9 % erhalten.

### Beispiel 4

Aus dem Filtrat, das bei der Durchführung von Beispiel 1 erhalten wird, wird das Acetonitril abdestilliert. Zu dem Destillationsrückstand werden 282,5 g Phenol (3 mol), 180 g 9-Fluorenon (1 mol) und 0,5 g Mercaptopropionsäure hinzugefügt. Anschließend wird die Kondensationsreaktion wie in Beispiel 1 beschrieben durchgeführt.

Bei der erneuten Produktabtrennung kann restliches, nicht isoliertes Fluorenderivat aus der ersten Reaktion mit abgetrennt werden, so daß sich die Ausbeute auf 88 % der Theorie erhöht.

### Beispiel 5

Der im Beispiel 1 erhaltene Destillationsrückstand wird anstelle von Acetonitril mit 500 ml Propionitril unter Rühren aufgelöst, auf - 15 °C abgekühlt und ausgefällt. Das abfiltrierte Addukt wird viermal mit je 150 ml Propionitril gewaschen.

### Beipiel 6

Wie in Beispiel 2 beschrieben, wird das im Beispiel 5 erhaltene Propionitril-Addukt entsprechend behandelt und 9,9-Bis-(4-hydroxyphenyl-) fluoren in 70 %iger Ausbeute der Theorie mit einer Reinheit von 98 % erhalten. Wird das Addukt vor der Isolierung noch einmal aus Propionitril umkristallisiert, erhält man ein 99,7 %iges Produkt.

### Beispiel 7

9,9-Bis-(4-hydroxyphenyl-) fluoren wird aus dem in Beispiel 5 beschriebenen Propionitril-Addukt freigesetzt, indem die erhaltenen weißen Kristalle im Vakuum bei 110 bis 120 °C getrocknet werden. 9,9-Bis-(4-hydroxyphenyl-) fluoren wird auf diese Weise in einer Ausbeute von 70 % der Theorie mit einer Reinheit von 98 % erhalten.

### Beispiel 8

In eine Schmelze von 180 g 9-Fluorenon (1 mol), 565 g Phenol (6 mol) und 1 g β-Mercaptopropionsäure werden bei 55 °C innerhalb von 6 h 14 g Chlorwasserstoff (0,38 mol) eingeleitet und noch 2 h bei 55 °C nachgerührt.

Anschließend wird das gebildete Reaktionswasser gemeinsam mit der enthaltenen Salzsäure abdestilliert. Der Destillationsrückstand wird unter Rühren in 500 ml Essigsäure aufgelöst. Bei ca. 100 °C werden 59,5 g (0,55 mol) Adipinsäuredinitril zugegeben und anschließend bis auf 15 °C abgekühlt. Dabei fällt ein weißes kristallines Produkt aus. Es handelt sich um ein Addukt aus 9,9-Bis-(4-hydroxyphenyl-) fluoren und Adipinsäure-dinitril im Molverhältnis von 2:1. Nach dem Abfiltrieren wird dieses Addukt noch zweimal mit je 150 ml Essigsäure und zweimal mit je 150 ml Toluol nachgewaschen. Nach dem Trocknen bei 60 °C im Vakuum werden 350 g des 9,9-Bis-(4-hydroxyphenyl-) fluoren -Adipinsäuredinitriladdukts erhalten. Dieses entspricht 86,5 % der Theorie.

### Beispiel 9

Die Herstellung von 9,9-Bis-(4-hydroxyphenyl-) fluoren sowie die Isolierung als Adipinsäuredinitril-Addukt erfolgt wie im Beispiel 8 beschrieben, anstelle von 500 ml Essigsäure werden jedoch 500 ml Essigsäureisobutylester eingesetzt. Die erhaltene Lösung wird aber auf 0 °C abgekühlt, das ausgefallene Addukt abgetrennt und gewaschen. Nach Trocknung bei ca. 60 °C im Vakuum werden 340 g (84 % der Theorie) Addukt erhalten.

### Beispiel 10

Die Herstellung von 9,9-Bis-(4-hydroxyphenyl-) fluoren sowie die Isolierung als Adipinsäuredinitril-Addukt erfolgt wie im Beispiel 8 und 9 beschrieben, anstelle von 500 ml Essigsäure werden 500 ml sek-Butanol eingesetzt. Die erhaltene Lösung wird auf 18 °C abgekühlt, das ausgefallene Addukt abgetrennt, gewaschen und bei ca. 60 °C im Vakuum getrocknet. Es werden 365 g (90,2 % der Theorie) Dinitriladdukt erhalten.

### Beispiel 11

Die Herstellung von 9,9-Bis-(4-hydroxyphenyl-) fluoren erfolgt wie im Beispiel 8 beschrieben. Nach erfolgter Reaktion werden anstelle von Adipinsäuredinitril 47 g Fumarsäuredinitril verwendet. Nach der Isolierung und dem Trocknen werden 276 g des 9,9-Bis-(4-hydroxyphenyl-) fluoren - Fumarsäuredinitriladduktes (2:1 mol) erhalten, was einer Ausbeute von 71 % der Theorie entspricht.

### Beispiel 12

Die Herstellung von 9,9-Bis-(4-hydroxyphenyl-) fluoren erfolgt wie im Beispiel 8 beschrieben. Nach erfolgter Reaktion werden anstelle von Adipinsäuredinitril 59 g Glutarsäuredinitril zugegeben. Nach der Isolierung und dem Trocknen werden 288 g des 9,9-Bis-(4-hydroxyphenyl-) fluoren - Glutarsäuredinitriladduktes (2:1 mol) erhalten, was einer 72,4 %igen Ausbeute der Theorie entspricht.

### Beispeil 13

Die Herstellung von 9,9-Bis-(4-hydroxyphenyl-) fluoren erfolgt wie im Beispiel 8 angegeben. Nach erfolgter Reaktion werden anstelle von Adipinsäuredinitril 80 g Oktansäuredinitril zugegeben. Nach der Isolierung und dem Trocknen werden 315 g 9,9-Bis-(4-hydroxyphenyl-) fluoren - Oktansäuredinitriladduktes erhalten. Das entspricht einer Ausbeute von 75,2 % der Theorie.

## Patentansprüche

1. Verfahren zur Herstellung von 9,9-Bis-(4-hydroxyphenyl-)fluoren durch eine bei 30 bis 90 °C durchgeführte und durch β-Mercaptopropionsäure und HCl-Gas katalysierte Kondensationsreaktion von Fluorenon und Phenol, die in molaren Verhältnissen von 1 : 4 bis 1 : 8 vorgelegt werden, **dadurch gekennzeichnet**, daß nach beendeter Reaktion entstandenes Reaktionswasser gemeinsam mit der gelösten Salzsäure abdestilliert wird, der Destillationsrückstand in einem Nitril gelöst wird, das entstehende auskristallisierende Produkt abgetrennt und in 9,9-Bis-(4-hydroxyphenyl-)fluoren und Nitril gespalten wird.

2. Verfahren gemäß Anspruch 1, **dadurch gekennzeichnet**, daß 9,9-Bis-(4-hydroxyphenyl-)fluoren durch thermische Behandlung vom Nitril getrennt wird.

3. Verfahren gemäß Anspruch 1. **dadurch gekennzeichnet**, daß 9,9-Bis-(4-hydroxyphenyl-)fluoren durch Aufschlämmen mit Wasser und ggf. Erwärmen vom Nitril getrennt und als weißes, kristallines Produkt abgetrennt wird.

4. Verfahrem gemäß Anspruch 1, **dadurch gekennzeichnet**, daß das 9,9-Bis-(4-hydroxyphenyl-)fluoren erst während der sich anschließenden Polymerisationsreaktion freigesetzt wird.

5. Verfahren gemäß der Ansprüche 1 bis 4, **dadurch gekennzeichnet**, daß als Nitril eine Verbindung der allgemeinen Formel R-CN verwendet wird, wobei R ein verzweigter oder unverzweigter, gesättigter Alkyl-, Aryl- oder Aralkylrest mit 1 bis 15 C-Atomen oder ein Arylalkylether ist.

6. Verfahren gemaß der Ansprüche 1 bis 4, **dadurch gekennzeichnet**, daß das Nitril eine Verbindung der allgemeinen Formel NC-A-CN ist, wobei A eine verzweigte oder unverzweigte, gesättigte Alkylkette mit 1 bis 6 C-Atomen oder eine substituierte oder unsubstituierte Arylgruppe ist.

7. Verfahren gemäß der Ansprüche 1 bis 4, **dadurch gekennzeichnet**, daß als Nitril Acetonitril verwendet wird.

8. Verfahren gemäß der Ansprüche 1 bis 4, **dadurch gekennzeichnet**, daß als Nitril Propionsäurenitril verwendet wird.

9. Verfahren gemäß der Ansprüche 1 bis 4, **dadurch gekennzeichnet**, daß als Nitril Adipinsäuredinitril verwendet wird.

10. Verfahren gemäß der Ansprüche 1 bis 9, **dadurch gekennzeichnet**, daß nach dem Abdestillieren des Reaktionswassers und der Salzsäure der Destillationsrückstand in der zwei- bis fünffachen molaren Menge Nitril, bezogen auf die eingesetzte Menge Fluorenon, gelöst wird.

## Claims

1. A process for the preparation of 9,9-bis(4-hydroxyphenyl)fluorene by means of a condensation reaction between fluorenone and phenol in molar ratios of 1:4 to 1:8 carried out at 30°C to 90°C and catalysed by β-mercaptopropionic acid and HCl gas, characterised in that, when the reaction is complete, reaction water which has formed is distilled off together with the dissolved hydrochloric acid, the distillation residue is dissolved in a nitrile, and the crystallising product which forms is separated off and separated into 9,9-bis(4-hydroxyphenyl)fluorene and nitrile.

2. A process according to claim 1, characterised in that 9,9-bis(4-hydroxyphenyl)fluorene is separated from the nitrile by heat-treatment.

3. A process according to claim 1, characterised in that 9,9-bis(4-hydroxyphenyl)fluorene is separated from the nitrile by suspension in water and, optionally, heating, and is separated as a white, crystalline product.

4. A process according to claim 1, characterised in that the 9,9-bis(4-hydroxyphenyl)fluorene is only released during the subsequent polymerisation reaction.

5. A process according to claims 1 to 4, characterised in that a compound of the general formula R-CN is used as a nitrile, where R is a branched or unbranched, saturated alkyl, aryl or aralkyl residue with 1 to 15 C atoms or is an arylalkyl ether.

6. A process according to claims 1 to 4, characterised in that the nitrile is a compound of the general formula NC-A-CN, where A is a branched or unbranched, saturated alkyl chain with 1 to 6 C atoms or is a substituted or unsubstituted aryl group.

7. A process according to claims 1 to 4, characterised in that acetonitrile is used as a nitrile.

8. A process according to claims 1 to 4, characterised in that propionitrile is used as a nitrile.

9. A process according to claims 1 to 4, characterised in that adiponitrile is used as a nitrile.

10. A process according to claims 1 to 9, characterised in that, after the reaction water and the hydrochloric acid have been distilled off, the distillation residue is dissolved in two to five times the molar quantity of nitrile in relation to the quantity of fluorenone used.

## Revendications

1. Procédé pour la préparation de 9,9-bis-(4-hydroxyphényl)-fluorène par une réaction de condensation de la fluorénone et du phénol menée à 30 jusqu'à 90°C et catalysée par l'acide β-mercaptopropionique et du gaz HCl, fluorénone et phénol qui sont mélangés dans des rapports molaires de 1:4 jusqu'à 1:8, procédé caractérisé en ce que, lorsque la réaction est achevée, l'eau réactionnelle résultante est séparée par distillation conjointement avec l'acide chlorhydrique dissous, et le résidu de distillation est dissous dans un nitrile, le produit obtenu se recristallisant étant séparé et divisé en 9,9-bis-(4-hydroxyphényl)-fluorène et en nitrile.

2. Procédé selon la revendication 1, caractérisé en ce que le 9,9-bis-(4-hydroxyphényl)-fluorène est séparé du nitrile par traitement thermique.

3. Procédé selon la revendication 1, caractérisé en ce que le 9,9-bis-(4-hydroxyphényl)-fluorène est séparé du nitrile par mise en suspension avec de l'eau et éventuellement par réchauffage et il est séparé sous forme de produit cristallin blanc.

4. Procédé selon la revendication 1, caractérisé en ce que le 9,9-bis-(4-hydroxyphényl)-fluorène n'est libéré que pendant la réaction de polymérisation consécutive.

5. Procédé selon les revendications 1 à 4, caractérisé en ce qu'en tant que nitrile, on met en oeuvre un composé de la formule R-CN, R étant un reste alkyle, aryle ou aralkyle saturé, ramifié ou non ramifié, avec 1 à 15 atomes C, ou un arylalkyléther.

6. Procédé selon les revendications 1 à 4, caractérisé en ce que le nitrile est un composé de la formule générale NC-A-CN, A étant une chaîne alkyle saturée, ramifiée ou non ramifiée, avec 1 jusqu'à 6 atomes C, ou un groupe aryle substitué ou non substitué.

7. Procédé selon les revendications 1 à 4, caractérisé en ce qu'en tant que nitrile, on utilise l'acétonitrile.

8. Procédé selon les revendications 1 à 4, caractérisé en ce qu'en tant que nitrile, on utilise le nitrile de l'acide propionique.

9. Procédé selon les revendications 1 à 4, caractérisé en ce qu'en tant que nitrile, on utilise le dinitrile de l'acide adipique.

10. Procédé selon les revendications 1 à 9, caractérisé en ce qu'après la séparation par distillation de l'eau de réaction et de l'acide chlorhydrique, le résidu de distillation est dissous dans la quantité molaire de nitrile de 2 à 5 fois par rapport à la quantité mise en ouevre de fluorénone.
